# EUROPEAN PATENT APPLICATION

(11) **EP 3 418 748 A1**
(43) Date of publication of application: **26.12.2018**
(21) Application number: 16890645.1
(22) Date of filing: 16.11.2016
(51) Int. Cl.: G01N 33/86, G01N 11/00, G01N 27/22

(54) **PLATELET AGGREGATION ACTIVITY ANALYSIS DEVICE, PLATELET AGGREGATION ACTIVITY ANALYSIS SYSTEM, PLATELET AGGREGATION ACTIVITY ANALYSIS PROGRAM, AND PLATELET AGGREGATION ACTIVITY ANALYSIS METHOD**

(30) Priority: 17.02.2016 JP 2016027938
(71) Applicant: Sony Corporation, Tokyo 108-0075 (JP)
(72) Inventor: MURATA Aya, Tokyo 108-0075 (JP); HAYASHI Yoshihito, Tokyo 108-0075 (JP); MACHIDA Kenzo, Tokyo 108-0075 (JP); BRUN Marcaurele, Tokyo 108-0075 (JP)
(74) Representative: D Young & Co LLP
(86) International application number: PCT/JP2016/083896
(87) International publication number: WO 2017/141508

(57) **Abstract**

There are provided a new device and a new method for measurement of platelet aggregability which can be used for monitoring in an anti-platelet therapy.

A platelet aggregability analyzing device includes: a blood coagulation system analyzing section that analyzes platelet aggregability on the basis of measurement data on a coagulation process of a platelet-containing sample to which a platelet inducing substance has been added; and an output control section that controls output of a result of an analysis by the blood coagulation system analyzing section.

## Description

### [Technical Field]

The present invention relates to a platelet aggregability analyzing device, a platelet aggregability analyzing system, a platelet aggregability analyzing program, and a method of analyzing platelet aggregability.

### [Background Art]

Anticoagulation therapy and antiplatelet therapy performed in the past have been therapeutic methods indispensable to prevention of thrombosis, and their usefulness has been verified by large-scale clinical tests. However, the effect of the antiplatelet therapy on reducing arterial thrombosis is lower than the brain infarction reducing effect of the anticoagulation therapy. One of the reasons for this is considered to lie in that in the anticoagulation therapy, the drug effect is appropriately monitored on the basis of each subject by prothrombin time international normalized ratio (PT-INR) or thrombo-test, but on the other hand, a monitoring method in the antiplatelet therapy has not yet been established although it has been reported that considerable individual differences are present in regard of sensitivity to an antiplatelet agent and continuation of its effect. Therefore, if appropriate monitoring of the drug effect can be accomplished by an easily understandable method also in the antiplatelet therapy, it becomes means for searching for an appropriate method of using drugs in each case, and an enhancement of the therapeutic effect can be expected.

The most fundamental function of platelets is sticking (adhesion) and aggregation. Platelet aggregability for determining a mutually adhered state of platelets is most widely spread as a method for quantitatively measuring this fundamental phenomenon, and examples of the test method therefor include (1) a transmittance method, (2) an impedance method, and (3) a particle calculation method. Depending on the kind and concentration of an aggregation inducing substance used in these test methods, details of lowering and accentuation of the platelet function can be known. Specifically, the transmittance method is commonly used as routine tests.

The transmittance method of (1) above is a method of quantifying platelet aggregation with time by adding a platelet stimulating substance and utilizing a rise in transparency of platelet-rich plasma (PRP) attendant on aggregation of platelets in the platelet-rich plasma.

Examples of problems involved in this method include the following (i) to (iv):
(i) Since separation of plasma is indispensable, specimen treatment until measurement is complicated, the amount of PRP obtained varies depending on the conditions of centrifugation, and the recovery rate of platelets is not constant. In addition, at the time of a PRP separating operation, platelets high in density are precipitated together with red blood cells, and their aggregation behaviors cannot be observed;
(ii) Since the intensity of platelet aggregation (aggregation rate) depends on the number of platelets in PRP to some extent, the difference in absorbance between before and after aggregation is small and a slight change cannot be detected, in the case where the number of platelets is not more than 100,000 platelets/µl;
(iii) A test using whole blood and that using turbid plasma such as chyle plasma are impossible to perform; and
(iv) Correlation between formation of platelet aggregates and light transmittance is bad.

The impedance method of (2) above is a method of detecting aggregation of platelets as a variation in electric resistance between electrodes, whereby aggregability of platelets in whole blood can be observed, and, since a centrifuging operation is absent, the aggregation behavior of all the platelets can be observed.

Examples of problems involved in this method include the following (v) and (vi):
(v) An initial-stage decrease in electric resistance arises from the presence of red blood cells between the electrodes, and it is difficult to determine an initial state of platelet aggregation; and
(vi) The aggregation pattern is not stable, as compared to the transmittance method.

The particle calculation method of (3) above is a method of detecting the degree of aggregation by calculating the number of platelet aggregates or singular platelets not associated with formation of aggregates, by a Coulter counter.

Examples of problems involved in this method include the following (vii) to (ix):
(vii) The procedure is troublesome;
(viii) Variation with time cannot be recorded; and
(ix) Dissolution of platelets due to an aggregation inducing substance may be erroneously calculated as a decrease in the number of singular platelets.

Meanwhile, in the blood coagulation system analysis, in recent years, a method of measuring dielectric constant during a blood coagulation process has been devised as a technique making it possible to evaluate blood coagulation measurement easily and accurately (PTL 1) .

This technique is a method of filling a capacitor-shaped sample section including a pair of electrodes with blood, and impressing an alternating voltage thereon to measure variation in dielectric constant attendant on the coagulation process of blood. Here, the blood specimen is collected from a vein by using citric acid as an anticoagulant, and, immediately before the start of measurement, an aqueous calcium chloride solution is added to release the anticoagulating action of citric acid, thereby permitting a blood coagulation reaction to proceed. The thus obtained data is analyzed according to a predetermined algorithm, whereby parameters relevant to blood coagulation, such as blood coagulation time, can be obtained.

By the blood coagulation system analyzing device by the measurement of dielectric constant, information associated with the influence of platelets on coagulation can also be acquired.

For example, it is possible to perform blood coagulation system analysis of acquiring information associated with the coagulability of blood, based on variation generated in complex dielectric constant spectrum measured during the coagulation process of blood, by adding a substance for activating or inactivating platelets to the blood (PTL 2). In this blood coagulation system analyzing method, in the case where a platelet activating substance is used as the substance, information associated with the coagulability of platelets contained in an inactive state in blood can be acquired on the basis of variation generated in the complex dielectric constant spectrum attendant on the activation of platelets by the substance. In addition, in the case where a platelet inactivating substance is used as the substance, information associated with coagulation of platelets contained in an active state in blood can be acquired on the basis of variation generated in the complex dielectric constant spectrum attendant on the inactivation of platelets by the substance.

On the other hand, according to the conventional blood coagulability tests such as prothrombin time international normalized ratio (PT-INR) and activated partial thromboplastin time (APTT), it is substantially possible to evaluate only the risk of bleeding attendant on a lowering in blood coagulability by excessive administration of an anticoagulant, and it is impossible to evaluate the risk of thrombus attendant on accentuation of blood coagulability. In addition, in the existing platelet aggregability test using platelet-rich plasma (PRP), a centrifugal separation procedure is indispensable, platelets would be activated during the procedure, so that accurate test results cannot be obtained, and the operation is also troublesome.

Besides, the conventional blood coagulability test involves the following problems (x) and (xi):
(x) It is necessary to preliminarily set a shortening width Δtₛ (reference value), which serves as a reference, of blood coagulation time, by use of a sample (whole blood) having normal coagulability; and
(xi) The measurement time is long because an accelerating agent is not added, at the time of permitting a coagulation reaction to proceed (if an accelerating agent is added, the difference due to platelet function becomes invisible).

### [Citation List]

### [Patent Literature]

[PTL 1]
   JP 2010-181400A
[PTL 2]
   JP 2012-194087A
[PTL 3]
   WO 2015/159623 pamphlet

### [Summary]

### [Technical Problem]

The present inventors made extensive and intensive researches in order to provide a new measuring method and device for platelet aggregability which can be used for monitoring in antiplatelet therapy, for example. As a result of their researches, they have completed the present technology.

### [Solution to Problem]

According to the present technology, there is provided a platelet aggregability analyzing device including:
a blood coagulation system analyzing section that analyzes platelet aggregability on the basis of measurement data on a coagulation process of a platelet-containing sample to which a platelet inducing substance has been added; and
an output control section that controls output of a result of an analysis by the blood coagulation system analyzing section.

The device may include a biosample holding section that holds the platelet-containing sample and the platelet inducing substance.

In addition, the device may include a measuring section that measures the coagulation process of the platelet-containing sample.

Further, the device may include a biosample supplying section that supplies the platelet-containing sample.

Furthermore, the device may include a drug supplying section that supplies the platelet inducing substance.

The blood coagulation system analyzing section may analyze the measurement data on a coagulation process of the platelet-containing sample to which the platelet inducing substance has been added, on the basis of measurement data on a coagulation process of a platelet-containing sample to which the platelet inducing substance has not been added.

Measurement information obtained by measurement of the coagulation process of the platelet-containing sample may be measurement data of an electrical characteristic and/or viscoelasticity.

The measurement data of the electrical characteristic may be dielectric constant of the platelet-containing sample, and the measurement data of the viscoelasticity may be measurement data obtained on the platelet-containing sample by a rheometer.

In addition, the platelet-containing sample is blood or plasma.

Further, the blood or plasma may be collected from a subject having been administered an anti-platelet-aggregation agent or an anticoagulant.

The platelet-containing sample and the platelet inducing substance are reacted with each other in a time of one to 20 minutes.

In addition, recovery of platelet aggregates may be conducted after the reaction between the platelet-containing sample and the platelet inducing substance.

According to the present technology, there is provided a platelet aggregability analyzing system including:
a platelet aggregability analyzing device including
   a blood coagulation system analyzing section that analyzes platelet aggregability on the basis of measurement data on a coagulation process of a platelet-containing sample to which a platelet inducing substance has been added, and
   an output control section that controls output of a result of an analysis by the blood coagulation system analyzing section; and
a display device that displays the result of the analysis.

According to the present technology, there is provided a platelet aggregability analyzing program for a computer to execute the steps of:
analyzing platelet aggregability on the basis of measurement data on a coagulation process of a platelet-containing sample to which a platelet inducing substance has been added; and
controlling output of a result of an analysis.

According to the present technology, there is provided a method of analyzing platelet aggregability, including the steps of:
analyzing platelet aggregability on the basis of measurement data on a coagulation process of a platelet-containing sample to which a platelet inducing substance has been added; and
controlling output of a result of an analysis.

### [Brief Description of Drawings]

[FIG. 1]

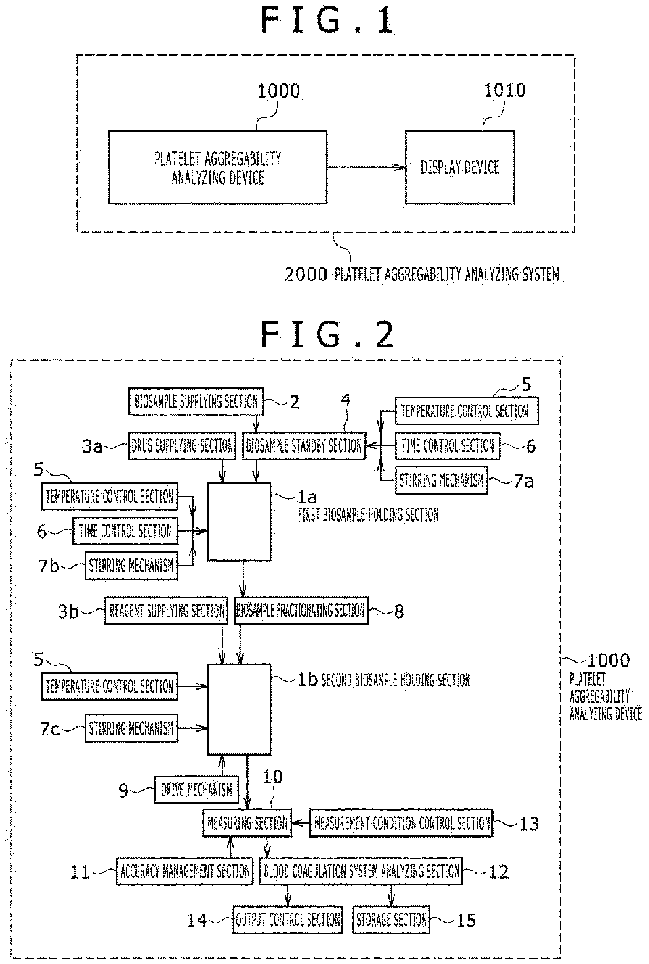

   FIG. 1 is a diagram depicting the configuration of a platelet aggregability analyzing system.
[FIG. 2]
   FIG. 2 is a diagram depicting the configuration of a platelet aggregability analyzing device.
[FIG. 3]

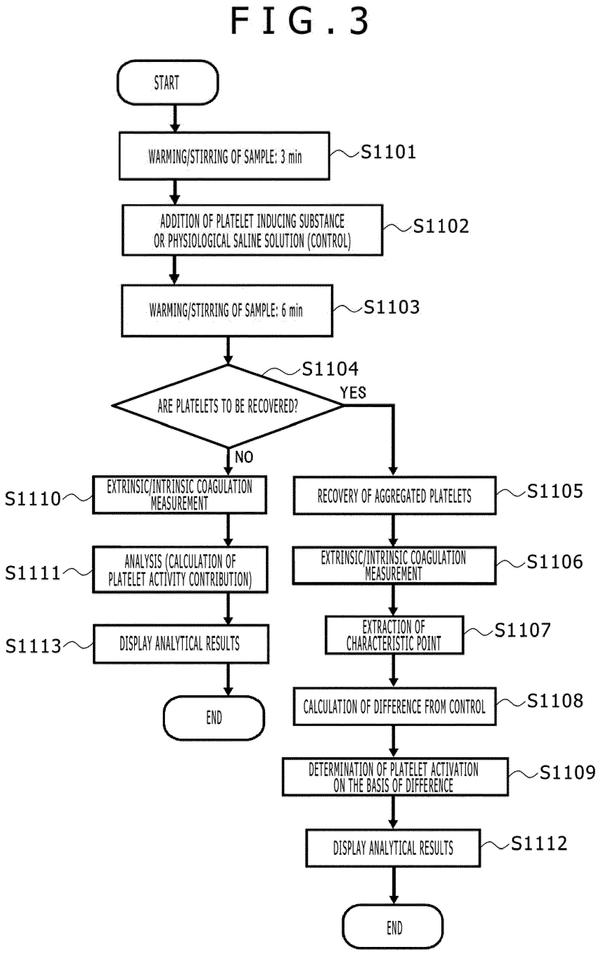

   FIG. 3 is a chart depicting steps of analysis of platelet aggregability.
[FIG. 4]

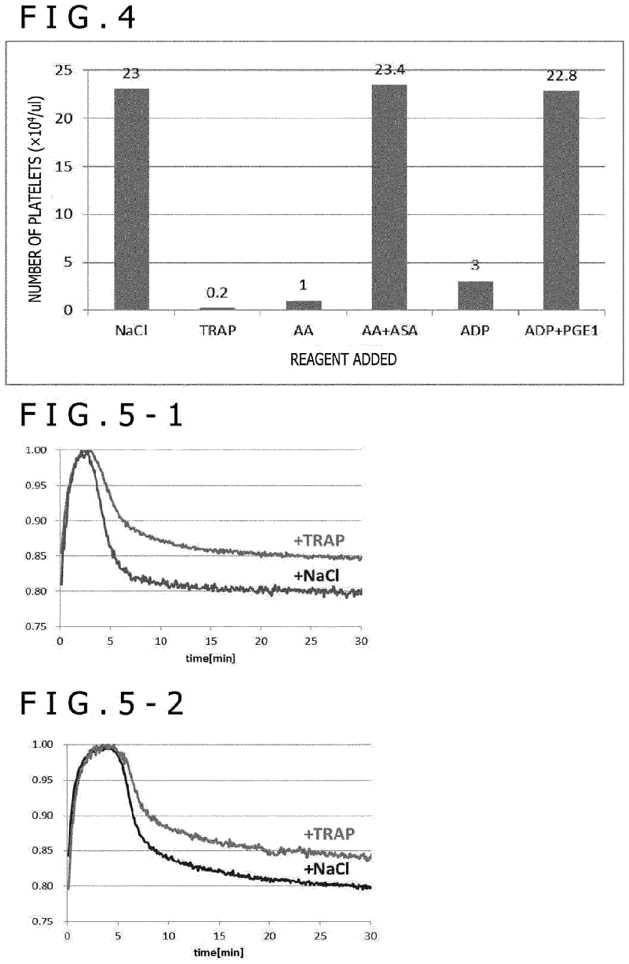

   FIG. 4 is a graph depicting variation in the number of platelets by addition of a platelet inducing substance to blood.
[FIG. 5-1]
   FIG. 5-1 is a graph depicting results of measurement of whole blood by a platelet aggregability analyzing device using dielectric constant.
[FIG. 5-2]
   FIG. 5-2 is a graph depicting results of measurement of whole blood by the platelet aggregability analyzing device using dielectric constant.
[FIG. 6-1]

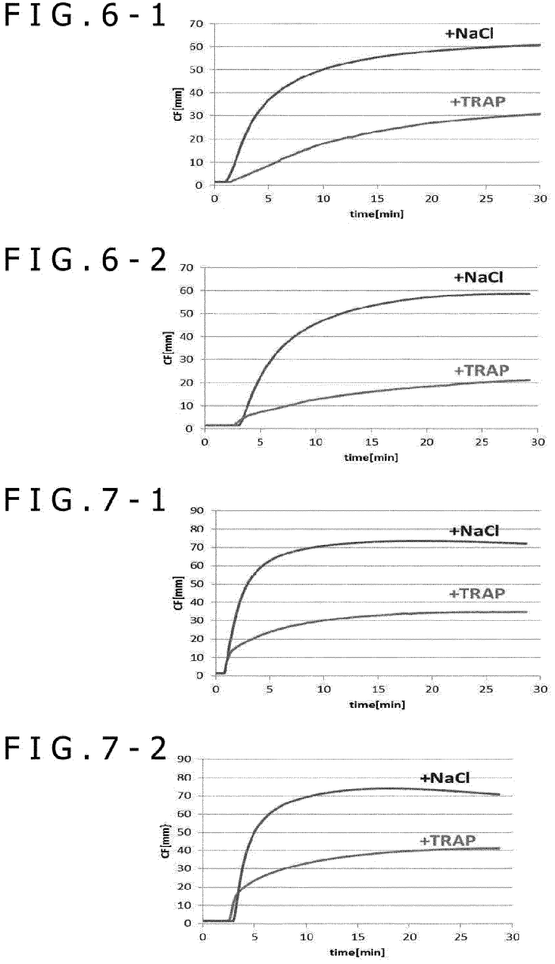

   FIG. 6-1 is a graph depicting results of measurement of whole blood by EXTEM of ROTEM.
[FIG. 6-2]
   FIG. 6-2 is a graph depicting results of measurement of whole blood by INTEM of ROTEM.
[FIG. 7-1]
   FIG. 7-1 is a graph depicting results of measurement of plasma by EXTEM of ROTEM.
[FIG. 7-2]
   FIG. 7-2 is a graph depicting results of measurement of plasma by INTEM of ROTEM.
[FIG. 8]

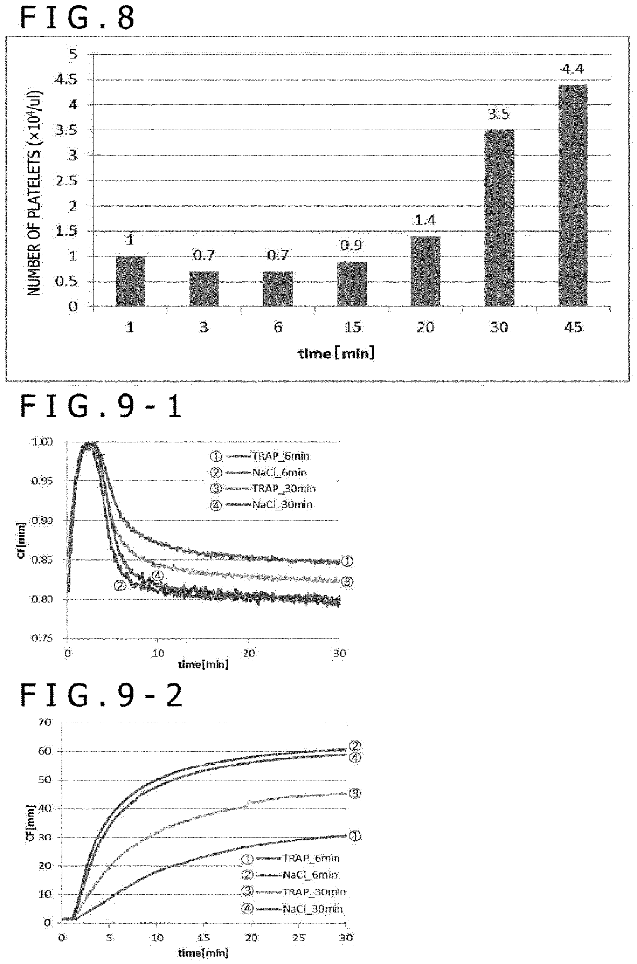

   FIG. 8 is a graph depicting variation in the number of platelets with reaction time after addition of a platelet inducing substance to whole blood.
[FIG. 9-1]
   FIG. 9-1 is a graph depicting results of measurement of whole blood by a platelet aggregability analyzing device using dielectric constant.
[FIG. 9-2]
   FIG. 9-2 is a graph depicting results of measurement of whole blood by EXTEM of ROTEM.
[FIG. 10-1]

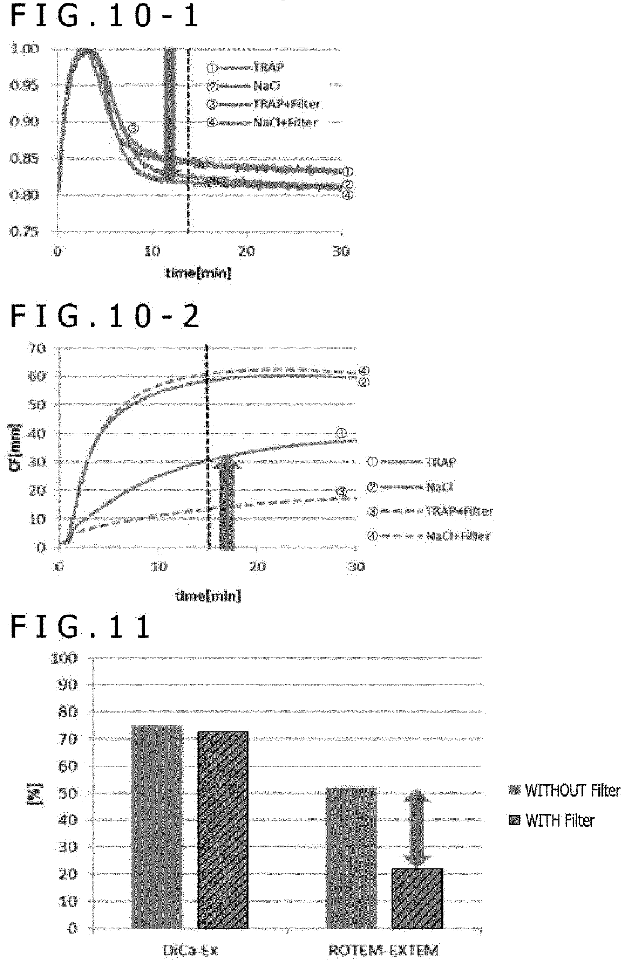

   FIG. 10-1 is a graph depicting results of measurement of whole blood by a platelet aggregability analyzing device using dielectric constant.
[FIG. 10-2]
   FIG. 10-2 is a graph depicting results of measurement of whole blood by EXTEM of ROTEM.
[FIG. 11]
   FIG. 11 is a graph depicting an effect of a filter.
[FIG. 12-1]

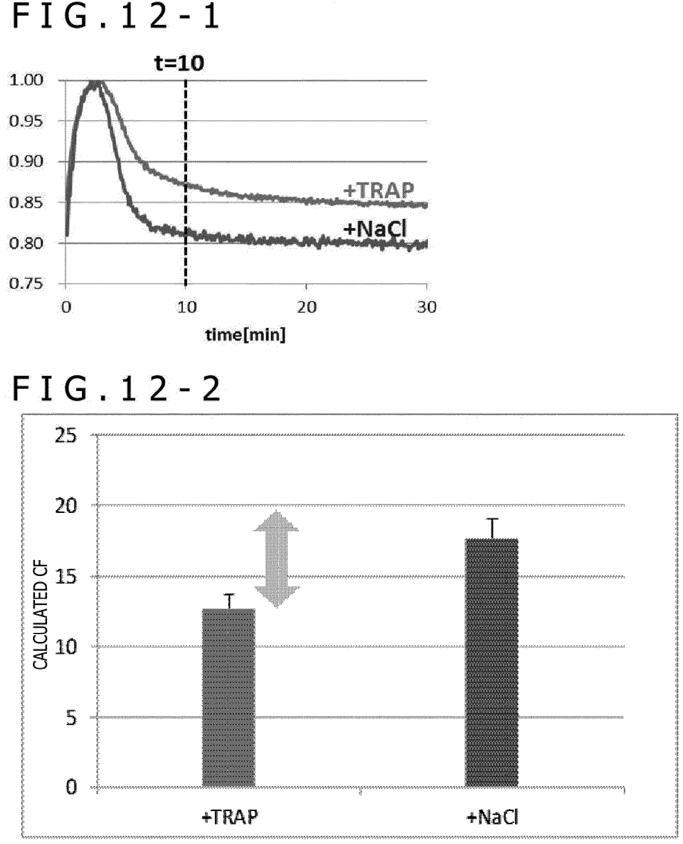

   FIG. 12-1 is a graph depicting results of measurement of whole blood by a platelet aggregability analyzing device using dielectric constant, wherein a maximum value is taken as 1.
[FIG. 12-2]
   FIG. 12-2 is a graph depicting results of measurement of whole blood by a platelet aggregability analyzing device using dielectric constant, wherein the reciprocal of a difference is calculated.

### [Description of Embodiment]

A preferred mode for carrying out the present technology will be described below. Note that the embodiment described below depicts a representative embodiment of the present technology, and the scope of the present technology is not to be construed narrowly thereby. Note that the description will be made in the following order.
1. Platelet aggregability analyzing system
2. Platelet aggregability analyzing device
   2-1. Configuration of the device
   2-2. Operation of the device
3. Embodiment
   3-1. Outline of the present embodiment
   3-2. Confirmation of occurrence of platelet aggregation
   3-3. Case where specimen is whole blood
   3-4. Case where specimen is plasma
   3-5. Effect of time of reaction between platelets and inducing substance
   3-6. Case of stimulation of platelets for long time
   3-7. Recovery of aggregated platelets by filter or the like
   3-8. Analyzing method for platelet aggregability
   3-9. Summary

### 1. Platelet aggregability analyzing system

A platelet aggregability analyzing system according to the present technology includes:
a platelet aggregating analyzing device including
   a blood coagulation system analyzing section that analyzes platelet aggregability on the basis of measurement data on a coagulation process of a platelet-containing sample to which a platelet inducing substance has been added, and
   an output control section that controls output of a result of an analysis by the blood coagulation system analyzing section; and
a display device that displays the result of the analysis.

FIG. 1 depicts the configuration of the platelet aggregability analyzing system.

A platelet aggregability analyzing system 2000 includes a platelet aggregability analyzing device 1000 and a display device 1010.

The platelet aggregability analyzing system 2000 measures platelet aggregability of a platelet-containing sample, and analyzes the platelet aggregability from the measurement data.

The display device 1010 displays the result of the analysis of the platelet aggregability.

The platelet aggregability analyzing device 1000 may include a computer or the like provided with a program for executing the platelet aggregability from the measurement data, and the display device 1010 may be a display or the like with which the computer is provided.

In addition, the platelet aggregability analyzing device 1000 may be able to analyze not only the platelet aggregability of the platelet-containing sample but also other blood coagulation system measurement items. Examples of the other blood coagulation system measurement items include coagulation of blood (blood clotting), fibrin formation, fibrin clot formation, blood clot formation, nummulation of red blood cells, blood aggregation, precipitation of red blood cells (blood sedimentation), clot retraction, hemolysis, and fibrinolysis. At the time of analyzing these items, an analyzing program for these items is used in place of the program for executing the platelet aggregability with which the computer is provided.

The display device 1010 may include an alarm section. In the alarm section, normal-value ranges for state changes of blood are preliminarily set, and the alarm section issues an alarm when an analytical result of a sample falls outside of the normal-value range.

The method of issuing an alarm is not particularly limited, and an alarm may be issued, for example, by a display or by a voice or sound.

### 2. Platelet aggregability analyzing device

### 2-1. Configuration of the device

FIG. 2 depicts a detailed configuration of an example of the platelet aggregability analyzing device 1000 described in FIG. 1 above. The platelet aggregability analyzing device 1000 includes a blood coagulation system analyzing section 12 and an output control section 14; more specifically, it also includes a biosample supplying section 2, a drug supplying section 3a, a reagent supplying section 3b, biosample holding sections 1a and 1b, and a measuring section 10 as main sections.

The biosample supplying section 2 supplies the platelet aggregability analyzing device 1000 with blood collected from a human or mammal.

The method of supplying the blood is not particularly limited; the blood may be supplied from a syringe, may be placed in a sample cartridge to be set on the platelet aggregability analyzing device 1000, or may be supplied directly from a subject to the platelet aggregability analyzing device 1000 through a tube. The subject may have undergone administration of an anti-platelet-aggregation agent or an anticoagulant.

A biosample standby section 4 permits blood fed from the biosample supplying section 2 to be maintained at a predetermined temperature and in a predetermined state. The biosample standby section 4 includes a temperature control section 5 that maintains the blood temperature, and a time control section 6 that controls a standby time of the blood, and, preferably, further includes a stirring mechanism 7a.

A first biosample holding section 1a supplies the aforementioned platelet inducing substance to the blood, allowing a reaction therebetween. The drug supplying section 3a is connected to the first biosample holding section 1a.

The drug supplying section 3a supplies the platelet inducing substance to the blood.

In addition, the first biosample holding section 1a includes a temperature control section 5, a time control section 6 and a stirring mechanism 7b. In the first biosample holding section 1a, the blood fed from the biosample standby section 4 is stirred with its temperature maintained, and platelets in the blood and the platelet inducing substance from the drug supplying section 3a are reacted with each other for a predetermined time.

A biosample fractionating section 8 fractionates the blood having undergone reaction with the platelet inducing substance in the first biosample holding section. The fractionated blood is sent from the biosample fractionating section 8 to a second biosample holding section 1b.

The second biosample holding section 1b supplies a coagulation accelerating substance to the blood, allowing a reaction therebetween. The reagent supplying section 3b is connected to the second biosample holding section 1b.

The reagent supplying section 3b supplies the coagulation accelerating substance to the blood.

Examples of the coagulation accelerating substance include calcium, extrinsic coagulation accelerating reagents, and intrinsic coagulation accelerating substances. One or a combination of two or more of these coagulation accelerating substances may be used to accelerate coagulation. Specific examples of the extrinsic coagulation accelerating reagents include tissue thromboplastin, and tissue factors. Specific examples of the intrinsic coagulation accelerating reagents include ellagic acid and kaolin.

Besides, the second biosample holding section 1b includes a temperature control section 5 and a stirring mechanism 7c, and may further include a drive mechanism 9. The drive mechanism 9 performs operations concerning the second biosample holding section 1b, such as driving of the temperature control section 5 and the stirring mechanism 7c, and feeding of blood.

In the second biosample holding section 1b, the blood and the coagulation accelerating substance from the reagent supplying section 3b are reacted with each other, with the blood kept at a predetermined temperature.

The measuring section 10 measures dielectric constant after or simultaneously with the addition of the coagulation accelerating substance to the blood. A rheometer may be used as the measuring section 10. Examples of the rheometer include a rotation thromboelastometry, thromboelastography, and ReoRox (tradename).

A measurement condition control section 13 adjusts temperature, measurement time and the like to conditions suitable for respective measuring methods. At the time of measuring dielectric constant at the measuring section 10, the measurement condition control section 13 controls, for example, a frequency, a measurement interval and/or the like used for the measurement.

In addition, an accuracy management section 11 performs management of data such as to prevent differences between measurements, variations in background or the like from being generated, monitoring of the state of each section of the device, etc., and this may be provided in the measuring section 10.

Note that a platelet recovery section provided with a filter or the like for recovery of the aggregated platelets may be provided upstream of the measuring section 10.

The blood coagulation system analyzing section 12 detects and analyzes a change in the state of the blood, on the basis of measurement data of dielectric constant. The blood coagulation system analyzing section 12 outputs the results of analysis to the output control section 14. The output control section 14 outputs the analytical results to the display device 1010, to display the analytical results. In addition, the blood coagulation system analyzing section 12 communicates with a storage section 15, which stores data of continuous measurement of a platelet-containing sample, time variations in analytical results, or previously measured data or analytical results, etc.

In the blood aggregability analyzing device 1000 described above, a processor executes a program stored in a memory or other recording medium, thereby causing the platelet aggregability analyzing device 1000 to perform various logical functions.

### 2-2. Operation of the device

First, blood is collected from a human or mammal. Whole blood may be used as it is as a sample, or the blood may be drawn, for example, at 1,000 rpm for 10 minutes to recover platelets and plasma, thereby obtaining a sample.

FIG. 3 depicts steps of analysis of platelet aggregability.

The sample is first supplied to the biosample supplying section 2. The sample is sent from the biosample supplying section 2 to the biosample standby section 4, where it is warmed to 37°C, is stirred, for example, for three minutes, and maintained (S1101). Next, the sample is sent to the biosample holding section 10, then a physiological saline solution is added to a control, whereas a platelet inducing substance is added to the sample to be measured (S1102). The platelet inducing substance to be added is not particularly limited, and there may be used collagen, epinephrine, ristocetin, calcium ions, thrombin, thromboxane A₂, thrombin receptive activated protein (TRAP), adenosine diphosphoric acid (ADP), arachidonic acid (AA), serotonin, adrenaline, noradrenaline or the like.

The concentration of the platelet inducing substance is preferably 1 to 100 µM more preferably 3 to 80 µM, and further preferably 5.4 to 62 µM in the case of thrombin receptive activated protein (TRAP). The concentration is preferably 0.1 to 100 µM, more preferably 0.5 to 30 µM, and further preferably 1.0 to 12.5 µM in the case of adenosine diphosphoric acid (ADP). The concentration is preferably 0.01 to 10 mM, more preferably 0.05 to 5.0 mM, and further preferably 0.08 to 1.0 mM in the case of arachidonic acid (AA).

In the first biosample holding section 1a, the physiological saline solution or the platelet inducing substance is added to the sample, then, in the first biosample holding section 1a, warming to 37°C is continued, and stirring is conducted, for example, preferably one to 20 minutes, more preferably three to six minutes (S1103), to consume the platelets. When stirring is conducted for not less than one minute, the reaction between the platelets and the platelet inducing substance proceeds, and when stirring is performed for not more than 20 minutes, collapse of platelet aggregates may not be reached.

Next, it is decided whether the consumed (aggregated) platelets are to be recovered (S1104). The recovery can be carried out by, for example, filtration (S1105).

The sample deprived of the aggregated platelets after it is decided that the aggregated platelets are to be recovered is served to extrinsic/intrinsic coagulation measurement in the measuring section 10 provided at the second biosample holding section 1b (S1106).

Subsequently, the blood coagulation system analyzing section 12 performs an analysis based on extrinsic/intrinsic coagulation measurement data in the following order.

First, the blood coagulation system analyzing section 12 performs extraction of a characteristic point, on the basis of the measurement results (S1107).

Examples of the characteristic point include a point at which measurement results of the control and the sample started to be stable, and a point at which a coagulation time of platelets is determined.

Next, the blood coagulation system analyzing section 12 calculates the difference between the control and the sample at the extracted characteristic point (S1108), and outputs the calculation result.

The blood coagulation system analyzing section 12 determines that the platelets have been activated more as the difference between the control and the sample at the characteristic point is greater (S1109).

Subsequently, the blood coagulation system analyzing section 12 outputs the analytical results to the display control section 14, which outputs the analytical results to the display device 1010, to display the analytical results (S1112).

Alternatively, in the case where it is decided that the aggregated platelets are not to be recovered, the aggregated platelets are not recovered, and extrinsic/intrinsic coagulation measurement is conducted in the measuring section provided at the second biosample holding section (S1110).

Next, an analysis of the contribution of platelets to coagulation is conducted (S1111). The analysis can be carried out by comparing the measurement result of the control to which the physiological saline solution has been added and the measurement result of the sample to which the platelet inducing substance has been added.

For example, by converting the measurement data obtained with lapse of time into a graph and checking the difference or ratio of measured values at a specific time or the difference or ratio of graph areas or the like, the contribution of platelets to coagulation can be analyzed. In the case where the difference between the sample and the control put to measurement is reduced, it is supposed that the platelet aggregating function is lowered or that a platelet inhibitor has been taken.

Subsequently, the blood coagulation system analyzing section 12 outputs the analytical results to the display control section 14, which outputs the analytical results to the display device 1010, to display the analytical results (S1113).

Note that the procedure depicted in the flow chart in FIG. 3 includes not only a process carried out on a time series basis along the described order but also processes which are not necessarily performed on a time series basis but are carried out in parallel or individually. Besides, in the case of a process which is carried out on a time series basis, the order can naturally be changed as required.

In addition, for carrying out the procedure, it is possible to produce a computer program to be incorporated in the hardware incorporated in the platelet aggregability analyzing device 1000 of the present technology. A recording medium in which the computer program is stored is also provided by the present technology.

While the above description is mainly of a technology (see JP 5691168, and JP 5768422) which utilizes a method of measuring a coagulation process by utilizing an electric characteristic (dielectric constant), measurement of a coagulation process can be conducted also by other methods.

As an example of the other methods, measurement of a coagulation process by viscoelasticity may be mentioned, and this method can be applied to the present technology.

For measurement of viscoelasticity, there can be used the above-mentioned thromboelastography (TEG (registered trademark)) blood coagulation analyzing device (Haemonetics Corporation), rotation thromboelastometry (ROTEM (registered trademark)) blood coagulation analyzing device (Finggal Link Co., Ltd.), and the like.

In the thromboelastography, a whole blood specimen is poured into a cup serving as a measurement vessel, an inducing substance according to the purpose of testing is added thereto, a rod-shaped pin hung by a wire is immersed in the specimen from above the vessel, and a steady reciprocating angular motion (typically a motion of reciprocating through a range of 4.45° in 10 seconds) is given to the vessel. Attendant on the progress of a coagulation reaction, the viscoelasticity of the specimen increases, the relative motion of the cup and the pin is reduced, and, therefore, rotation displacement of the pin increases. The rotation displacement is recorded with lapse of time by use of an optical system in the device, whereby a waveform called thromboelastogram is obtained.

The rotation thromboelastometry is based on basically the same principle, except that the reciprocating angular motion is given not to the cup but to the pin. While the above-mentioned prothrombin time or activated partial thromboplastin time is a coagulation end point detection method, the thromboelastography and thromboelastometry have the merit that a series of process from the start of coagulation to thrombus formation, and further to the subsequent fibrinolysis, can be monitored with lapse of time by a single device.

In the case of measuring a coagulation process by the above-mentioned rotation thromboelastometry, both whole blood and plasma can be used as the platelet-containing sample. In the case of using the platelet aggregability analyzing device 1000, the use of whole blood is preferred.

### 3. Embodiment

An exemplary embodiment will be described below, but the present technology is not limited thereto.

### 3-1. Outline of the present embodiment

First, a platelet inducing substance was added to whole blood or platelet-rich plasma (PRP), followed by stirring for a predetermined time, whereon the platelets responded an aggregation reaction, and the degree of the reaction was confirmed by a reduction in the number of platelets. The number of platelets was measured by a multi-item automated blood cell counter pocH-100i (Sysmex Corporation) in which the principle of measurement is the DC detection method of classifying the kinds of cells according to the cell size.

### 3-2. Confirmation of occurrence of platelet aggregation

### <Experimental Method>

Collection of venous blood from a healthy subject was conducted by an ordinary method using commercialized vacuum blood collection tubes (blood collection amount: 1.8 mL, five tubes) enclosing citric acid as an anticoagulant. The first tube of blood was discarded without being put to use, and the remaining four tubes of blood were put to the following experiment. In addition, the blood was put to use after left to stand at room temperature for approximately 30 minutes after the blood collection.

First, 300 uL of whole blood was stirred at 37°C for three minutes. Thereafter, a platelet inducing substance was added to the whole blood, and a reaction was allowed for six minutes. In this instance, a system with a physiological saline solution added to the whole blood was prepared as a control, and the number of platelets in the blood after the reaction was measured by a multi-item automated blood cell counter pocH-100i (Sysmex Corporation).

Besides, for confirming reproducibility, a plurality of runs of experiment were conducted, and, further, similar experiments for other healthy subjects than the above were also performed.

### <Results>

FIG. 4 depicts the number of platelets in blood used as a control and the numbers of platelets when platelet inducing substances were added to blood. In FIG. 4, NaCl described as an added reagent refers to a physiological saline solution, and the system in that case was used as the control. In addition, TRAP refers to thrombin receptive activated protein, AA refers to arachidonic acid, AA+ASA refers to arachidonic acid and aspirin, ADP refers to adenosine diphosphoric acid, and ADP+PGE1 refers to adenosine diphosphoric acid and prostaglandin E1.

As depicted in FIG. 4, it was found that the number of platelets is decreased by addition of a platelet inducing substance. From the results, it was found that the addition of a platelet inducing substance increased the number of platelet aggregates in the blood and decreased the number of singular platelets.

As a substance for activating platelets, there can be used thrombin receptive activated protein (TRAP), adenosine diphosphoric acid (ADP), arachidonic acid (AA), collagen, epinephrine, ristocetin, thromboxane A₂ (TAX₂), adrenaline, etc.; in the present embodiment, TRAP, AA, and ADP were used.

In addition, measurement of the number of platelets was conducted also in the cases where a substance for inactivating the respective activation routes was added.

As a result, it was found that in the case where an inactivating substance is added, even if an activating agent is added the number of platelet aggregates would not increase, and singular platelets would remain as they are.

### 3-3. Case where specimen is whole blood

### <Experimental Method>

Measurement of blood coagulability was conducted using the blood put to reaction in the experiment of 3-2 above. The measurement was carried out using a blood coagulation system analyzing device (dielectric coagulometer prototype No. 2), for performing the measurement of dielectric constant of the sample as aforementioned, and a comprehensive hemostatic ability measuring system ROTEM (registered trademark), at a measurement temperature of 37°C.

In the case of the blood coagulation system analyzing device, measurement of DiCA-Ex to which an aqueous calcium solution (0.2 M Ca) and a commercialized extrinsic stimulus reagent (100 fold dilution + PBS (v/v = 1/5)) were added immediately before the start of measurement, and measurement of DiCA-In to which an aqueous calcium solution (0.2 M Ca) and a commercialized intrinsic stimulus reagent (+ PBS (v/v = 1/5)) were added, were conducted. The amount was unifiedly set to be 12 uL per 180 uL of blood.

In ROTEM, measurement of EXTEM based on a principle of adding tissue thromboplastin or a tissue factor as a coagulant, and measurement of INTEM based on a principle of adding ellagic acid as an activating agent and adding partial thromboplastin, were performed.

Besides, for confirming reproducibility, a plurality of runs of experiment were conducted, and, further, similar experiments for other healthy subjects than the above were also performed.

### <Results>

As depicted in FIGS. 5-1 and 5-2, when time variation in dielectric constant (normalized) at 100 kHz after the addition of a physiological saline solution (control; +NaCl) was compared with that after the addition of a platelet inducing substance (+TRAP), it was found that the amplitude is raised when the platelet inducing substance is added.

In ROTEM, also, as depicted in FIG. 6-1 (EXTEM) and FIG. 6-2 (INTEM), variations in CF representing the hardness or firmness of blood clot appeared.

The variation is due to consumption of platelets, and it was found that platelet aggregability can be measured by similar method also in ROTEM (FIGS. 6-1 and 6-2) .

### 3-4. Case where specimen is plasma

### <Experimental Method>

Collection of venous blood from a healthy subject was conducted by an ordinary method using commercialized vacuum blood collection tubes (blood collection amount: 1.8 mL, three tubes) enclosing citric acid as an anticoagulant. The first tube of blood was discarded without being put to use, and the remaining two tubes of blood were put to the following experiment. In addition, after left to stand at room temperature for approximately 30 minutes after the blood collection, the two tubes of blood were centrifuged at 1,000 rpm for 10 minutes, and platelet-rich plasma (PRP) was recovered.

First, 300 uL of plasma was stirred at 37°C for three minutes. Thereafter, 20 uL of a platelet inducing substance was added, and a reaction was allowed for six minutes. In this instance, a system with a physiological saline solution added to the plasma was prepared as a control, and the number of platelets in the blood after the reaction was measured by a multi-item automated blood cell counter pocH-100i (Sysmex Corporation). Measurements of EXTEM and INTEM were performed by ROTEM. Note that in ROTEM, plasma can also be measured as a specimen.

### <Results>

As depicted in FIGS. 7-1 and 7-2, variations in CF representing the hardness or firmness of blood clot appeared, like in the case of whole blood. Accordingly, it was depicted that platelet-containing plasma and whole blood can be used as the specimen to be measured.

### 3-5. Effect of time of reaction between platelets and inducing substance

### <Experimental Method>

Using whole blood (collected by the method similar to the experiment of 3-2 above), an experiment concerning the reaction time (warming/stirring time) after the addition of a platelet inducing substance was conducted. After the platelet inducing substance was added to blood, variation in the number of platelets after each of reaction times (one to 45 minutes) was measured by pocH-100i (Sysmex Corporation).

### <Experimental Results>

As depicted in FIG. 8, it was found that the number of platelets decreased to a minimum in three to six minutes, and thereafter the number is recovered with the lapse of time. Therefore, it is supposed that the function of platelets is most activated after three to six minutes of the addition of the platelet inducing substance, and it can be decided that the influence on the coagulation reaction by the platelet aggregation is most clearly observable after this reaction time.

Note that though not depicted in FIG. 8, the number of platelets of the blood used as a specimen in this experiment in the case where the inducing substance was not used was 340,000 platelets/uL.

In addition, the lower limit for normal number of platelets is 50,000 platelets/uL. Therefore, it is assumed that after 45 minutes of the addition of the platelet inducing substance and then on, it becomes difficult to observe the variation in coagulability due to variation in platelet aggregation. For confirming this, the following experiment of 3-6 was conducted.

### 3-6. Case of stimulation of platelets for long time

### <Experimental Method>

Collection of venous blood from a healthy subject was conducted by an ordinary method using commercialized vacuum blood collection tubes (blood collection amount: 1.8 mL, five tubes) enclosing citric acid as an anticoagulant. The first tube of blood was discarded without being put to use, and the remaining four tubes of blood were put to the following experiment. In addition, the blood was put to use after left to stand at room temperature for approximately 30 minutes after the blood collection.

First, 300 uL of whole blood was stirred at 37°C for three minutes. Thereafter, 20 uL of a platelet inducing substance was added to the blood, and a reaction was allowed for six minutes or for 30 minutes. Using the blood having undergone the reaction, measurement of coagulability was conducted. The device and reagent concentration used for the measurement were similar to those in the experiment of 4-3.

In this instance, a system with a physiological saline solution added to the blood in the same amount as that of the platelet inducing substance was also put to measurement as a control.

### <Results>

As depicted in FIGS. 9-1 and 9-2, it was found that when warming/stirring was conducted for 30 minutes after the addition of the platelet inducing substance, the measurement result approaches to that of a system obtained without addition of the platelet inducing substance. In other words, the experimental results of 4-5 were confirmed.

### 3-7. Recovery of aggregated platelets by filter or the like

### <Experimental Method>

After platelets were activated by adding a platelet inducing substance to whole blood in the manner similar to the above-described experimental methods, the whole blood after the reaction was passed through a filter (PluriStrainer), then measurement of DiCa-Ex was conducted by a blood coagulation system analyzing device, and measurement of EXTEM was performed by ROTEM.

The filter size used was 15 to 25 um, which is the size of small aggregates of platelets. In this experiment, the results obtained when the filter size was 15 um are depicted.

### <Results>

In the case of DiCa-Ex, as depicted in FIG. 10-1, no large difference was observed between the system passed through the filter and the system not passed through the filter. Specifically, the system in which platelets were present as aggregates and the system from which aggregates were removed by the filter depicted substantially the same ratio. Therefore, it is supposed that the presence of aggregates does not influence on the observation of the difference from the control.

On the other hand, in the case of ROTEM-EXTEM, as depicted in FIG. 10-2, the presence/absence of removal of platelet aggregates by use of the filter produced a change in the effect of the addition of the inducing substance. It was found that in the case of using ROTEM, the difference from the control is rather clearly observable when the platelet aggregates are physically removed before measurement of coagulation.

Note that other methods for removing the platelet aggregates include a method in which microbeads coated with an activated platelet-specific surface antibody are used before the start of measurement.

In FIG. 10-1 (DiCa-Ex) and FIG. 10-2 (ROTEM-EXTEM), the time of 15 minutes determinative of coagulation time was taken as an investigation time, and ratio of NaCl - TRAP (without filter) and NaCl+Filter - TRAP+Filter (with filter) in regard of the 15-minute value (DiCa is the difference between the maximum value of 1 and the 15-minute value) was calculated.

The results are depicted in FIG. 11.

In the case of DiCa-Ex, the presence/absence of the filter did not produce a large difference in the ratio; in the case of ROTEM, the difference in the ratio was larger when the filter was present than when the filter was absent.

### 3-8. Analyzing method for platelet aggregability

Based on the results of measurement by the platelet coagulation analyzing device using dielectric constant in 3-3 above, an example of the analyzing method will be depicted.

The time of 10 minutes determinative of coagulation time was taken as an investigation time (FIG. 12-1), the maximum value was taken as 1, and the reciprocal of the difference therefrom was calculated (calculated CF), the results being depicted in FIG. 12-2. An average value obtained in the case where NaCl was added (control) was 17.7, whereas an average value obtained in the case where the platelet inducing substance was added was 12.7 (n = 4).

The range indicated by the arrows in FIG. 12-2 is reflected by the platelet function (platelet contribution part), and it is assumed that this difference is reduced in the case where the platelet aggregation function has been lowered or in the case where a platelet inhibitor is taken. This was actually confirmed by a combination of ADP with a prostaglandin E1 inhibitor (ADP route inhibitor) and the like.

Besides, since p = 0.001 (p < 0.01) in the t-test, it could be decided that there is a significant difference between the average value obtained with addition of NaCl and the average value obtained with addition of a platelet inducing substance.
(Here, in the t-distribution, if the value falls within the outside 5% range, it is considered that the samples are not from the same population and a significant difference is present therebetween, with p being the probability of falling outside the 95% confidence interval.)

Other than the above-mentioned analyzing method, there may be considered a method in which the difference from a control is calculated from the areas, or the ratio, as to a graph formed from coagulation process measurement data obtained. Since the blood coagulation system analyzing device offers various manners of evaluation of measurement data according also to measurement frequency, it is high in the degree of freedom as compared to ROTEM, and there is a possibility that other parameters than the platelet function can also be calculated simultaneously.

In addition, in the present technology, an analyzing method can be carried out which includes the steps of:
(1) obtaining measurement data on a coagulation process of a platelet-containing sample to which a platelet inducing substance or the like has not been added (data representing an aggregating effect of platelets and fibrin can be obtained);
(2) obtaining, on the other hand, measurement data on a coagulation process of a platelet-containing sample to which a platelet function inhibitor has been added (data (of fibrin) on removal of platelets can be obtained); and (3) obtaining, further, measurement data on a coagulation process of a platelet-containing sample to which a platelet coagulation inducing substance has been added. In the case where the subject is taking an antiplatelet agent, the result of measurement appears between data of (1) and data of (2), depending on the degree of its effect.

### 3-9. Summary

In accordance with the present technology, a blood coagulation system analyzing device can be used as a platelet aggregability analyzing device not only for measurement concerning a coagulation factor but also for measurement of a platelet function, which can lead to a monitoring therapy on the basis of the kind of the antiplatelet agent.

In addition, according to the present technology, measurement using whole blood, which is a better sample, can be carried out as a clinical test with thrombosis in mind.

Note that the present technology can assume the following configurations.
<1>
   A platelet aggregability analyzing device including:
   a blood coagulation system analyzing section that analyzes platelet aggregability on the basis of measurement data on a coagulation process of a platelet-containing sample to which a platelet inducing substance has been added; and
   an output control section that controls output of a result of an analysis by the blood coagulation system analyzing section.
<2>
   The platelet aggregability analyzing device as described in the above paragraph <1>, further including:
   a biosample holding section that holds the platelet-containing sample and the platelet inducing substance.
<3>
   The platelet aggregability analyzing device as described in the above paragraph <1> or <2>, further including:
   a measuring section that measures the coagulation process of the platelet-containing sample.
<4>
   The platelet aggregability analyzing device as described in any one of the above paragraphs <1> to <3>, further including:
   a biosample supplying section that supplies the platelet-containing sample.
<5>
   The platelet aggregability analyzing device as described in any one of the above paragraphs <1> to <4>, further including:
   a drug supplying section that supplies the platelet inducing substance.
<6>
   The platelet aggregability analyzing device as described in any one of the above paragraphs <1> to <5>, in which the blood coagulation system analyzing section analyzes measurement data on a coagulation process of the platelet-containing sample to which the platelet inducing substance has been added, on the basis of measurement data on a coagulation process of a platelet-containing sample to which the platelet inducing substance has not been added.
<7>
   The platelet aggregability analyzing device as described in any one of the above paragraphs <1> to <6>, in which measurement information obtained by measurement of the coagulation process of the platelet-containing sample is measurement data of an electrical characteristic and/or viscoelasticity.
<8>
   The platelet aggregability analyzing device as described in the above paragraph <7>, in which the measurement data of the electric characteristic is dielectric constant of the platelet-containing sample.
<9>
   The platelet aggregability analyzing device as described in the above paragraph <7>, in which the measurement data of the viscoelasticity is measurement data obtained on the platelet-containing sample by a rheometer.
<10>
   The platelet aggregability analyzing device as described in any one of the above paragraphs <1> to <9>, in which the platelet-containing sample is blood or plasma.
<11>
   The platelet aggregability analyzing device as described in the above paragraph <10>, in which the blood or plasma is collected from a subject having been administered an anti-platelet-aggregation agent or an anticoagul.
<12>
   The platelet aggregability analyzing device as described in any one of the above paragraphs <1> to <11>, in which the platelet-containing sample and the platelet inducing substance are reacted with each other in a time of one to 20 minutes.
<13>
   The platelet aggregability analyzing device as described in the above paragraph <12>, in which recovery of platelet aggregates is conducted after the reaction between the platelet-containing sample and the platelet inducing substance.
<14>
   A platelet aggregability analyzing system including:
   a platelet aggregability analyzing device including
   a blood coagulation system analyzing section that analyzes platelet aggregability on the basis of measurement data on a coagulation process of a platelet-containing sample to which a platelet inducing substance has been added, and
   an output control section that controls output of a result of an analysis by the blood coagulation system analyzing section; and
   a display device that displays the result of the analysis.
<15>
   A platelet aggregability analyzing program for a computer to execute the functions of:
   analyzing platelet aggregability on the basis of measurement data on a coagulation process of a platelet-containing sample to which a platelet inducing substance has been added; and
   controlling output of a result of an analysis.
<16>
   A method of analyzing platelet aggregability, including the steps of:
   analyzing platelet aggregability on the basis of measurement data on a coagulation process of a platelet-containing sample to which a platelet inducing substance has been added; and
   controlling output of a result of an analysis.

### [Reference Signs List]

1a First biosample holding section
1b Second biosample holding section
2 Biosample supplying section
3a Drug supplying section
3b Reagent supplying section
4 Biosample standby section
5 Temperature control section
6 Time control section
7a, 7b Stirring mechanism
8 Biosample fractionating section
9 Drive mechanism
10 Measuring section
11 Accuracy management section
12 Blood coagulation system analyzing section
13 Measurement condition control section
14 Output control section
15 Storage section
1000 Platelet aggregability analyzing device
1010 Display device
2000 Platelet aggregability analyzing system

## Claims

1. A platelet aggregability analyzing device comprising:
a blood coagulation system analyzing section that analyzes platelet aggregability on a basis of measurement data on a coagulation process of a platelet-containing sample to which a platelet inducing substance has been added; and
an output control section that controls output of a result of an analysis by the blood coagulation system analyzing section.

2. The platelet aggregability analyzing device according to claim 1, further comprising:
a biosample holding section that holds the platelet-containing sample and the platelet inducing substance.

3. The platelet aggregability analyzing device according to claim 1, further comprising:
a measuring section that measures the coagulation process of the platelet-containing sample.

4. The platelet aggregability analyzing device according to claim 1, further comprising:
a biosample supplying section that supplies the platelet-containing sample.

5. The platelet aggregability analyzing device according to claim 1, further comprising:
a drug supplying section that supplies the platelet inducing substance.

6. The platelet aggregability analyzing device according to claim 1, wherein the blood coagulation system analyzing section analyzes measurement data on a coagulation process of the platelet-containing sample to which the platelet inducing substance has been added, on a basis of measurement data on a coagulation process of a platelet-containing sample to which the platelet inducing substance has not been added.

7. The platelet aggregability analyzing device according to claim 1, wherein measurement information obtained by measurement of the coagulation process of the platelet-containing sample is measurement data of an electrical characteristic and/or viscoelasticity.

8. The platelet aggregability analyzing device according to claim 7, wherein the measurement data of the electrical characteristic is dielectric constant of the platelet-containing sample.

9. The platelet aggregability analyzing device according to claim 7, wherein the measurement data of the viscoelasticity is measurement data obtained on the platelet-containing sample by a rheometer.

10. The platelet aggregability analyzing device according to claim 1, wherein the platelet-containing sample is blood or plasma.

11. The platelet aggregability analyzing device according to claim 10, wherein the blood or plasma is collected from a subject having been administered an anti-platelet-aggregation agent or an anticoagulant.

12. The platelet aggregability analyzing device according to claim 1, wherein the platelet-containing sample and the platelet inducing substance are reacted with each other in a time of one to 20 minutes.

13. The platelet aggregability analyzing device according to claim 12, wherein recovery of platelet aggregates is conducted after the reaction between the platelet-containing sample and the platelet inducing substance.

14. A platelet aggregability analyzing system comprising:
a platelet aggregability analyzing device including
a blood coagulation system analyzing section that analyzes platelet aggregability on a basis of measurement data on a coagulation process of a platelet-containing sample to which a platelet inducing substance has been added, and
an output control section that controls output of a result of an analysis by the blood coagulation system analyzing section; and
a display device that displays the result of the analysis.

15. A platelet aggregability analyzing program for a computer to execute the steps of:
analyzing platelet aggregability on a basis of measurement data on a coagulation process of a platelet-containing sample to which a platelet inducing substance has been added; and
controlling output of a result of an analysis.

16. A method of analyzing platelet aggregability, comprising the steps of:
analyzing platelet aggregability on a basis of measurement data on a coagulation process of a platelet-containing sample to which a platelet inducing substance has been added; and
controlling output of a result of an analysis.
